# FASCICULE DE BREVET EUROPEEN

(11) **EP 1 551 222 B1**
(45) Date de publication et mention de la délivrance du brevet: **01.07.2009**
(21) Numéro de dépôt: 03780222.0
(22) Date de dépôt: 14.10.2003
(51) Int. Cl.: A01K 67/027

(54) **PROCEDE POUR PRODUIRE UN MAMMIFERE RENDU RESISTANT A UNE INFECTION PAR UN ALPHA HERPES VIRUS AINSI QUE MAMMIFERE OBTENU PAR LA MISE EN OEUVRE DE CE PROCEDE ET DESCENDANT D'UN TEL MAMMIFERE**
VERFAHREN ZUR HERSTELLUNG VON ALPHA HERPES VIRUS RESISTENTEN SÄUGETIEREN, MIT DIESEM VERFAHREN HERGESTELLTE SÄUGETIERE SOWIE IHRE NACHKOMMEN
METHOD FOR PRODUCING A MAMMAL PROVIDED WITH RESISTANCE TO AN ALPHA-HERPES VIRUS MEDIATED INFECTION AND MAMMAL OBTAINED BY IMPLEMENTING SAID METHOD AND SAID MAMMAL'S PROGENY

(30) Priorité: 15.10.2002 FR 0212775
(43) Date de publication de la demande: 13.07.2005
(73) Titulaire: France Hybrides, 45110 Chateauneuf sur Loire (FR)
(72) Inventeur: ETSURO, Ono, Sapporo 063-0012 (JP); TOSHIMITSU, Uede, Sapporo 004-0835 (JP)
(74) Mandataire: Cabinet HERRBURGER
(86) Numéro de dépôt international: PCT/FR2003/003024
(87) Numéro de publication internationale: WO 2004/035775

(56) Documents cités:
- WO-A-01/79496
- WO-A-02/28902
- WO-A-98/25967
- US-A1- 2002 102 644
- MILNE R S ET AL: "Porcine HveC, a member of the highly conserved HveC/nectin 1 family, is a functional alphaherpesvirus receptor." VIROLOGY. UNITED STATES 15 MAR 2001, vol. 281, no. 2, 15 mars 2001 (2001-03-15), pages 315-328, XP002247513 ISSN: 0042-6822 cité dans la demande -& DATABASE SWISSPROT [Online] 16 octobre 2001 (2001-10-16), XP002247516 extrait de EBI Database accession no. Q9GL76
- DATABASE BIOSIS [Online] BIOSCIENCES INFORMATION SERVICE, PHILADELPHIA, PA, US; 7 mars 2001 (2001-03-07), INOBE MANABU ET AL: "Functional analysis of HVEM, a member of TNFR family, by using a transgenic mice expressing soluble form of HVEM." XP002248746 Database accession no. PREV200100276732 & FASEB JOURNAL, vol. 15, no. 4, 7 mars 2001 (2001-03-07), page A352, Annual Meeting of the Federation of American Societies for Experimental Biology on Experimental Biology 2001;Orlando, Florida, USA; March 31-April 04, 2001 ISSN: 0892-6638
- WANG J ET AL: "The regulation of T cell homeostasis and autoimmunity by T cell-derived LIGHT." THE JOURNAL OF CLINICAL INVESTIGATION. UNITED STATES DEC 2001, vol. 108, no. 12, décembre 2001 (2001-12), pages 1771-1780, XP002247515 ISSN: 0021-9738

## Description

On connaît différents virus de type herpès qui se distinguent par leur génome ainsi que par leurs caractéristiques biologiques.

Une sous famille de ces virus correspond aux alpha herpès virus parmi lesquels on peut nommer les virus herpès simplex humain de type 1 et de type 2 (HSV-1 et HSV-2), le virus de la maladie d'Aujeszky ou Pseudorabies virus (PRV) et l'herpès virus bovin de type 1 (BHV-1).

Tous ces virus présentent la particularité d'être neurotropes et d'avoir un cycle de réplication très bref et un large spectre d'hôte.

L'infection par ces virus provoque des lésions de l'épiderme, se situant en règle générale au niveau des muqueuses, suivies d'une propagation du virus au système nerveux pouvant y entraîner des inflammations aiguës, ainsi que des infections latentes.

Parmi les alpha herpès virus entraînant les ravages les plus importants, on peut citer le virus PRV qui est un agent pathogène d'une importance économique majeure en production porcine tant par le coût direct des pathologies induites que par celui des moyens de lutte mis en oeuvre.

Ce virus est largement présent dans la plupart des régions de forte production porcine (Europe, Amérique du Nord, Asie).

Il existe actuellement plusieurs vaccins contre le virus PRV qui représentent un marché important à l'échelle mondiale.

Une telle vaccination n'est cependant pas sans inconvénients compte tenu en particulier du coût entraîné par la nécessité de vacciner une large proportion des animaux d'un troupeau et les problèmes pratiques liés à cette nécessité qui rendent cette opération particulièrement incommode, ce d'autant plus qu'il est en général nécessaire de faire plusieurs injections.

Le coût et les contraintes de cette opération peuvent en limiter l'usage et par là même également l'efficacité.

Des stratégies d'éradication du virus sont également utilisées avec des résultats variables mais toujours fragiles.

Le virus BHV-1 responsable de la rhinotrachéite infectieuse bovine provoque lui aussi des ravages très importants dans les élevages.

Ce virus est en effet très contagieux tant chez les veaux nouveaux nés que chez les animaux plus âgés ; il peut provoquer une inflammation au niveau de la cavité nasale, du larynx, des lésions oculaires et des affections respiratoires, mais également se localiser au niveau du cerveau en provoquant des encéphalites ou encore prendre des formes génitales.

Ces différentes affections qui sont fréquemment fatales chez le veau nouveau né entraînent des pertes très importantes pour les éleveurs ; de plus, chez les vaches laitières, on peut observer une chute dramatique de la production de lait.

Il existe également plusieurs vaccins contre le virus BHV-1, mais la vaccination présente les mêmes inconvénients que la vaccination contre le virus PRV dans les élevages porcins.

De plus des vaccins contre le virus BHV-1 injectés par voie intra musculaire ont été considérés comme responsables d'avortements chez les vaches gestantes.

L'éradication du virus a également été proposée notamment dans certains pays européens, mais elle se révèle particulièrement difficile en raison du caractère latent du virus qui peut demeurer longtemps inactif au sein d'un organisme avant de se manifester, notamment sous l'effet d'un stress.

Par suite, la conception d'un procédé permettant d'engendrer des lignées de porcs transgéniques constitutivement résistants au virus PRV ou encore des lignées de bovins transgéniques constitutivement résistants au virus BHV-1 aurait un intérêt économique considérable.

L'invention a pour objet de proposer un tel procédé.

Celui-ci a pu être conçu grâce à des recherches préalables effectuées sur un modèle souris, qui tout comme le porc ou le bovin est sensible à certains alpha herpès virus et en particulier aux virus HSV1 et PRV.

Il est connu que les alpha herpès virus se lient aux cellules d'abord grâce à l'interaction d'une glycoprotéine virale gC, entrant dans la constitution de la membrane du virion et de l'héparane sulfate présent à la surface des cellules, alors que la fusion ultérieure entre l'enveloppe du virion et la membrane cellulaire fait, quant à elle, intervenir d'autres glycoprotéines (gB, gD, gH et gL).

De nombreux travaux ont porté sur l'étude de récepteurs potentiels des alpha herpès virus présents à la surface des cellules de mammifères hôtes ayant une capacité de fixation de la particule virale et pouvant éventuellement neutraliser ainsi son pouvoir infectieux.

Quatre protéines récepteurs des alpha herpès virus ont été identifiées à ce jour à savoir :
- le HVEM ou HveA qui est un médiateur d'entrée des virus HVS1 et HVS2 mais pas du virus PRV (Montgomery, R. I., Warner, M. S., Lum B.J., and Spear, P.G. (1996). Herpes simplex virus-1 entry into cells mediated by a novel member of the TNF/NGF receptor family. Cell 87, 427-436).
- trois membres de la superfamille des immunoglobulines (HveB ou nectin-2 ; HveC ou nectin-1 et HveD)
   (Cocchi, F., Menotti, L., Mirandola, P., Lopez, M., and Campadelli-Fiume, G. (1998). The ectodomain of a novel member of the immunoglobulin subfamily related to the poliovirus receptor has the attribute of a bona fide receptor for herpes simplex virus types 1 and 2 in human cells. J. Viral. 72, 9992-10002 ;
   Geraghty, R. J., Krummenacher, C., Eisenberg, R. J., Cohen G. H., and Spear, P. G. (1998) Entry of alphaherpesviruses mediated by poliovirus receptor related protein 1 and poliovirus receptor. Science 280, 1618-1620 ;
   Shukla, D., Liu, J., Blaiklock, P., Shworak, N. W., Bai, X., Esko, J. D., Cohen, G. H., Eisenberg, R. J., Rosenberg, R. D., and Spear, P. G. (1999). A novel role for 3-O-sulfate heparan sulfate in herpes simplex virus entry. Cell 99, 13-22 ;
   Warner, M. S., Martinez, W., Geraghty, R. J., Montgomery, R. I., Witbeck, J. C., Xu, R., Eisenberg, R ; J., Cohen, G. H., and Spear, P. G. (1998). A cell surface protein with herpesvirus entry activity (HveB) confers susceptibility to infection by herpes simplex virus type 2, mutants of herpes simplex virus type 1 ans pseudorabies virus. Virology 246, 179-189.

Selon les publications Spear, P. G. (1993). Entry of alphaherpesviruses into cells. Semin. Virol. 4, 167-180 ; Campadelli-Fiume, G., Arsenakis, M., Farabegoli, F., and Roizman, B. (1988). Entry of herpes simplex virus 1 in BJ cells that constitutively express viral glycoprotein D is by endocytosis and results in degradation of the virus. J. Virol. 62, 159-167, il a été suggéré que, outre la liaison initiale avec l'héparane sulfate, c'est l'interaction de la glycoprotéine gD de l'alpha herpès virus avec un récepteur présent à la surface de la cellule qui permet l'entrée du virus sous une forme infectieuse et que dans certains types de cellules, les virus HSV-1, PRV et BHV-1 peuvent utiliser un récepteur commun de la glycoprotéine gD pour entrer dans la cellule.

Il a ainsi été prouvé que la protéine HVEM peut permettre l'entrée des virus HSV1 et HSV2 dans des cellules non permissives, mais pas celle du virus PRV.

En revanche, il a été démontré que la protéine HveC, et notamment celle du porc se comporte comme un récepteur fonctionnel non seulement du virus HSV.1 mais également des alpha herpès virus animaux PRV et BHV-1 (Milne, R. S. B., Connolly, S. A., Krummenacher, C., Eisenberg, R. J., and Cohen, G. H. (2001). Porcine HveC, a member of the highly conserved HveC/nectin 1 family, is a functional alphaherpesvirus receptor. Virology 281, 315-328 ; Cocchi, F., Menotti, L., Mirandola, P., Lopez, M., and Campadelli-Fiume, G. (1998). The ectodomain of a novel member of the immunoglobulin subfamily related to the poliovirus receptor has the attribute of a bona fide receptor for herpes simplex virus types 1 and 2 in human cells. J. Virol. 72, 9992-10002 ; Geraghty, R. J., Krummenacher, C., Eisenberg, R. J., Cohen G. H., and Spear, P. G. (1998) Entry of alphaherpesviruses mediated by poliovirus receptor related protein 1 and poliovirus receptor. Science 280, 1618-1620).

Les capacités de liaison à la glycoprotéine virale gD sont plus particulièrement attribuées au domaine V pour HveC et aux deux premiers domaines riches en cystéine (« CRD ») pour HVEM (Structure-Based Analysis of the Herpes Simplex Virus Glycoprotein D Binding Site Present on Herpesvirus Entry Mediator HevA (HVEM). Connolly SA, Landsburg DJ, Carfi A, Wiley DC, Eisenberg RJ, Cohen GH ; J virol 2002, Nov 1 ; 76(21) : 10894-10904).

Cependant il a été montré dans la publication Martinez WM, Spear PG, Amino acid substitutions in the V domain of nectin-1 (HveC) that impair entry activity for herpes simplex virus types 1 and 2 but not for Pseudorabies virus or bovine herpesvirus 1, J Virol. 2002 Jul ; 76(14) : 7255-62, que les capacités de liaison avec la glyco protéine virale gD de la protéine HveC pouvaient être altérées significativement saris oblitérer les capacités de cette protéine à déclencher la fusion des membranes cellulaires et virales et permettre ainsi l'entrée des virus PRV et BHV-1 dans la cellule sous une forme infectieuse.

Il a également été montré dans la publication Campadelli-Fiume G, Cocchi F, Menotti L, Lopez M. the novel receptors that mediate the entry of herpes simplex viruses and animal alphaherpesviruses into cells Rev Med Virol. 2000 Sep-Oct ; 10(5) : 305-19, que la protéine HveC exprimée par la souris peut jouer un rôle de médiateur de l'entrée des alpha herpès virus PRV, BHV-1 et HSV indépendamment d'une interaction détectable avec la glycoprotéine gD.

Il a de surcroît globalement été conclu dans l'étude Geraghty RJ, Fridberg A, Krummenacher C, Cohen GH, Eisenberg RJ, Spear PG, use of chimeric nectin-1 (HveC)-related receptors to demonstrate that ability to bind alphaherpesvirus gD is not necessarily sufficient for viral entry, Virology. 2001 Jul 5 ; 285(2) : 366-75, que l'interaction de la protéine HveC avec la glycoprotéine gD n'était pas suffisante pour permettre l'entrée du virus dans la cellule et que les caractéristiques de la protéine HveC responsables de ses propriétés de médiateur de l'entrée des virus HSV, PVR et BHV1 dans les cellules cibles ne pouvaient être réduites à ses capacités de liaison avec la glycoprotéine gD.

Il est en outre à noter que la protéine HveC possède une séquence polypeptidique remarquablement bien conservée entre les espèces de mammifères ; à titre d'exemple, 97 % des acides aminés sont communs entre la protéine HveC exprimée par le porc et la protéine HveC exprimée chez les bovins, ce qui implique une forte identité de structure et de fonction chez ces deux espèces.

Selon la publication INOBE MANABU et al « Functional analysis of HVEM, a member of TNFR family, by using a transgenic mice expressing soluble form of HVEM » (Biosciences information Service, Philadelphia, PA - US - 2001.03.07), on a créé dans un but expérimental, des lignées de souris génétiquement modifiées, en introduisant dans le génome de ces souris un transgène codant pour une protéine chimérique constituée du domaine extracellulaire de la protéine HVEM et de la portion cristallisable Fc de l'immunoglobuline IgG, ce pour étudier l'implication de la protéine HVEM dans la régulation du système immunitaire.

Il a été ainsi confirmé que l'expression d'une forme soluble de HveM, membre de la famille du récepteur du TNFalpha, produit un effet immunosuppresseur.
A partir de ces connaissances préalables, il est proposé conformément à l'invention un procédé pour produire un mammifère appartenant à une espèce non humaine rendu résistant par transgénèse germinale à une infection par un alpha herpès virus, pour lequel le polypeptide HveC ou nectin-1 constitue un récepteur fonctionnel **caractérisé en ce qu**'on introduit par insertion ou recombinaison homologue dans le génome des cellules constituant la lignée germinale du mammifère, un transgène permettant l'expression d'une protéine chimérique constituée d'une part du domaine extracellulaire de la nectin-1 ou HveC c'est-à-dire des trois domaines protéiques VCC ou d'une forme tronquée de ce domaine extracellulaire consistant en le domaine V, et d'autre part du fragment cristallisable d'une immunoglobuline, dans un système d'expression approprié.

L'idée à la base de l'invention a donc consisté à utiliser partiellement les capacités de médiateur de la protéine HveC vis-à-vis de l'entrée du virus visé, mais ce d'une façon inoffensive pour la cellule (en isolant son domaine cellulaire ou une forme tronquée de ce domaine) de façon à inhiber finalement l'entrée de ce virus dans la cellule et à favoriser son élimination, ce par un processus qui reste encore à déterminer.

Le mécanisme d'action de la protéine chimérique exprimée pourrait notamment comprendre, grâce à la capacité de fixation de celle-ci à la fois pour la particule virale et le récepteur cellulaire Fc, une augmentation des capacités de phagocytose et de destruction des virions par les macrophages et cellules dendritiques et une activation des lymphocytes cytotoxiques NK.

Il pourrait également comprendre la formation de récepteurs membranaires des alpha herpès virus, sous la forme de multimères fonctionnellement altérés par incorporation d'une ou plusieurs unités de la protéine chimérique, permettant la fixation des particules virales à la surface de la cellule mais pas leur entrée dans le cytoplasme sous une forme infectieuse.

Selon l'invention, la protéine HveC ou nectin-1 et/ou l'immunoglobuline appartiennent de préférence à l'espèce homologue.

La première étape du procédé conforme à l'invention correspond donc à la préparation du transgène qui peut être effectuée en mettant en oeuvre des méthodes bien connues des spécialistes et abondamment décrites dans la littérature constituant à cloner :
D'une part, soit l'ADN complémentaire de l'ARN transcrit pour le gène du récepteur cellulaire, à partir d'une préparation d'ARN extraite d'un prélèvement de tissu pris sur un mammifère, soit la région chromosomique (ensemble des exons et introns) constituant le gène de ce récepteur à partir d'une préparation d'ADN génomique extraite également d'un prélèvement de tissu réalisé sur un mammifère, soit une construction chimérique constituée pour partie de l'ADNc et pour la partie restante de la chaîne polypeptidique du fragment génomique correspondant (« minigène »).

Dans tous les cas, on utilisera seulement la partie correspondant au domaine extracellulaire de ce récepteur c'est-à-dire les trois domaines protéiques VCC ou une forme tronquée de ce domaine extracellulaire consistant en le domaine V.

D'autre part, soit l'ADNc complémentaire de l'ARN transcrit pour l'un des gènes de chaîne lourde pour la classe et la sous-classe d'immunoglobuline choisie (par exemple G1), à partir d'une préparation d'ARN extraite d'un prélèvement de tissu pris sur un mammifère, soit la région chromosomique (ensemble des exons et introns) constituant ce gène de chaîne lourde à partir d'une préparation d'ADN génomique extraite également d'un prélèvement de tissu réalisé sur un mammifère, soit une construction chimérique constituée pour partie de l'ADNc et pour la partie restante de la chaîne polypeptidique du fragment génomique correspondant (« minigène »). La construction réalisée retiendra avantageusement les régions codant pour les domaines Hinge, CH₂ et CH₃ uniquement de la chaîne lourde d'immunoglobuline choisie (fraction cristallisable).

Un exemple d'une telle construction pour l'immunoglobuline humaine G1 est décrit dans la publication CTLA-4 Is a Second Receptor for the B Cell Activation Antigen B7. By Peter S. Linsley, William Brady, Mark Urnes, Laura S. Grosmaire, Nitin K. Damle, and Jeffrey A. Ledbetter ; J. Exp. Med. © The Rockefeller University Press ; volume 174, Septembre 1991, 561-569.

Les opérations de clonage seront réalisées à partir des connaissances préalables existantes relatives aux gènes utilisés, à savoir leur séquence, leur localisation chromosomique, ceci si possible dans l'espèce homologue, sinon en se basant sur les séquences connues pour ce gène chez d'autres mammifères.

Ces opérations pourront être réalisées par une réaction de polymérisation en chaîne (PCR) précédée d'une étape de transcription reverse pour l'ADN complémentaire ou par la détection au sein de banques d'ADN génomique de l'espèce visée des clones susceptibles de s'hybrider avec une sonde spécifique ou de produire un amplicon PCR spécifique du gène recherché.

Cette construction sera réalisée de façon à joindre les séquences codant pour le domaine extracellulaire de la nectin-1 ou HveC c'est-à-dire les trois domaines protéiques VCC ou pour une forme tronquée de ce domaine extracellulaire consistant en le domaine V, en 5' des séquences codant pour le fragment cristallisable de la chaîne lourde d'immunoglobuline (terminée par un codon stop) en respectant le cadre de lecture original des deux gènes, et éventuellement la nature et l'efficacité des jonctions introns-extrons s'ils ont été inclus, de façon à assurer, au final, l'expression d'une protéine chimérique constituée pour sa partie amino terminale du polypeptide correspondant au domaine extracellulaire du récepteur cellulaire HveC ou à l'une de ses sous parties et pour sa partie carboxy terminale des domaines Hinge, CH2 et CH3 de la chaîne lourde d'immunoglobuline.

Cette construction sera réalisée dans un vecteur d'expression permettant une expression forte de la protéine chimérique dans un ou plusieurs compartiments biologiques de l'hôte où elle permettra la protection des cellules de manière à rendre l'hôte globalement résistant à l'infection initiale ou à son développement. Le procédé consistera notamment à utiliser des systèmes d'expression actifs soit de façon constitutive dans l'ensemble des cellules soit plus spécifiquement dans des tissus cibles de l'infection virale tels les tissus du système nerveux central ou les tissus épithéliaux (notamment ceux du système respiratoire).

Le vecteur d'expression pourra comprendre une région promotrice, un signal de terminaison, des éléments stimulateurs de la transcription, des séquences isolatrices du contexte chromatinien, d'autres unités de transcription, tous éléments susceptibles d'assurer l'expression souhaitée.

Le procédé consistera avantageusement en l'emploi de systèmes d'expression constitués à partir de séquences régulatrices clonées chez l'espèce homologue, ou pour l'application à des animaux de rente, chez d'autres animaux d'élevage habituellement consommés par l'homme.

La seconde étape du procédé conforme à l'invention consiste à introduire le transgène ainsi obtenu dans le génome des cellules constituant la lignée germinale du mammifère hôte visé par insertion ou recombinaison homologue, là encore par une méthode bien connue des spécialistes de sorte que ce transgène s'intègre dans le patrimoine génétique de ce mammifère.

On pourra notamment utiliser à cette fin la micro injection pronucléaire du segment d'ADN encodant le transgène ou le transfert nucléaire de cellules transformées en culture par le transgène.

L'invention concerne également un mammifère appartenant à une espèce non humaine rendu résistant par transgénèse germinale à une infection par un alpha herpès virus pour lequel le polypeptide HveC ou nectin-1 constitue une récepteur fonctionnel par l'effet de l'expression d'une protéine chimérique constituée d'une part du domaine extracellulaire de la nectin-1 ou HveC de préférence de l'espèce homologue, et d'autre part du fragment cristallisable d'une immunoglobuline, notamment d'une immunoglobuline de type gamma de préférence de l'espèce homologue.

Selon l'invention, l'alpha herpès virus peut avantageusement être le virus PRV et le mammifère appartenir à l'espèce porcine.

Selon une variante de l'invention l'alpha herpès virus peut également être le virus BHV-1 et le mammifère appartenir à l'espèce bovine.

Il est essentiel conformément à l'invention que l'opération de transgénèse soit une transgénèse germinale de façon que les descendants du mammifère soient eux aussi susceptibles d'exprimer la protéine chimérique.

L'invention concerne ainsi un mammifère renfermant dans le génome de ses cellules, un transgène codant pour une protéine chimérique constituée d'une part du domaine extracellulaire de la nectin-1 ou HveC, c'est-à-dire des trois domaines proteiques VCC ou d'une forme tronquée de ce domaine extracellulaire consistant en le domaine V et d'autre part, du fragment cristallisable d'une immunoglobuline, dans un système d'expression approprié, ce transgène ayant été inséré dans le génome de la lignée germinale de l'un de ses ancètres.

L'invention se rapporte également à un matériel génétique tel que des semences ou des ovocytes ou des embryons essentiellement dérivés de mammifères transgéniques du type susmentionné.

Plusieurs exemples de séquences de protéines chimériques conformes à l'invention sont jointes en annexe.

Il s'agit de séquences en acides aminés qui comprennent le peptide signal à l'extrémité amino terminale qui sera processé lors de la maturation.

Une protéine chimérique conforme à ce modèle est également décrite dans le document JP-2001-328430 dans le cadre d'une autre application.

La faisabilité du procédé conforme à l'invention a été confirmée par
1) des résultats expérimentaux obtenus dans le cadre de l'analyse in vivo de la résistance au virus herpes simplex humain de type 1 (HSV-1) de souris transgéniques exprimant une protéine chimérique Hvem - Ig.

Selon ces tests on a introduit par transgénèse germinale dans l'ADN de ces souris un transgène codant pour une protéine chimérique constituée du domaine extracellulaire du récepteur murin HVEM de ce virus et de la portion cristallisable Fc de l'immunoglobuline IgG-1 humaine.

Le domaine extracellulaire murin HveM a été cloné par RT PCR à partir d'une préparation d'ARN extraite de cellules de rate stimulées par la concavaline A, obtenues sur des souris de souche BALB/c.

Les amorces utilisées pour la réaction RT PCR étaient 5'-TAACTCGAGCTCTTGGCCTGAAGTTTC-3' et 5'-TTAAGGATCCGAGGAGCAGGTGGTGTCT-3'

L'ADNc a été inséré dans les sites de restriction Xhol et BamHl d'un plasmide portant la séquence du fragment cristallisable de l'immunoglobuline G1 humaine (comme décrit dans la publication Nakagawa I., Murakami, M., Ijima, K., Chikuma, S., Saito, I., Kanegae, Y., Ishikura, H., Yoshiki, T., Okamoto, H., Kitabatake, A., and Uede, T. (1998). Persistent and secondary adenovirus-mediated hepatic gene expression using adenovirus vector containing CTLA41gG. Human Gene Therapy 9, 1739-1745).

Le fragment XhoI/XbaI contenant l'ADN encodant la protéine chimérique HveMIg a été isolé de cette construction et inséré à son tour, après mise en bouts francs des extrémités, dans le site de restriction SwaI du vecteur cosmidique pAxCAwt (distribué commercialement par la société TAKARA, Kyoto, Japon) sous le contrôle du promoteur CAG (promoteur de la β actin) connu pour permettre une expression constitutive élevée dans tout type de cellule (Niwa, H., Yamamura, K., and Miyazaki, J. (1991). Efficient selection for high-expression transfectants with a novel eukaryotic vector. Gene 108, 193-200).

On a créé ensuite par micro injection du fragment Pmel/Sall de ce vecteur, contenant le facteur de stimulation de la transcription du gène viral CMV IE, le promoteur du gène Beta actin de la poule, la séquence de la protéine HVEMIg et le signal de polyadénylation 3' du locus Beta globin de lapin, dans les pronucléi d'embryons fécondés de souris (génotype F1 C57 BL/6 X SJL) trois lignées A, B, C de souris transgéniques chacune issue d'un fondateur indépendant exprimant cette protéine chimérique HVEM Ig.

La présence de la protéine HVEMIg a été détectée comme bande spécifique révélée par un anticorps anti-HVEMIg (produit par hype-rimmunisation sur lapin) par immunoélectrophorèse dans le sérum des trois lignées de souris transgéniques, avec un titre en moyenne plus faible pour les souris de la lignée B.

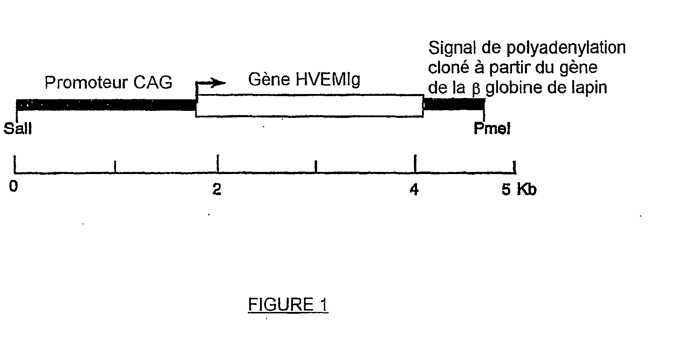

La construction effectuée est représentée schématiquement sur la figure 1.

La concentration en protéine chimérique HVEMIg du sérum des souris des trois lignées transgéniques A B C est représentée sur les tableaux 1, 2, 3 et 4 joints en annexe.

Les souris transgéniques de trois lignées se sont développées normalement et on n'a pas constaté de différences entre les poids de ces souris et ceux de leurs homologues non transgéniques de la même portée.

Pour déterminer si les souris transgéniques exprimant la protéine chimérique HVEMIg étaient effectivement protégées contre le virus HSV-1, on a inoculé par voie intraveineuse et une dose de 10⁹ ufc correspondant à dix fois la dose létale (10 DL 50) de virus, des souris transgéniques et des souris non transgéniques de contrôle de la même portée.

La DL 50 a été déterminée initialement chez la lignée de souris la moins sensible des deux ayant servi à la production des animaux transgéniques hybrides.

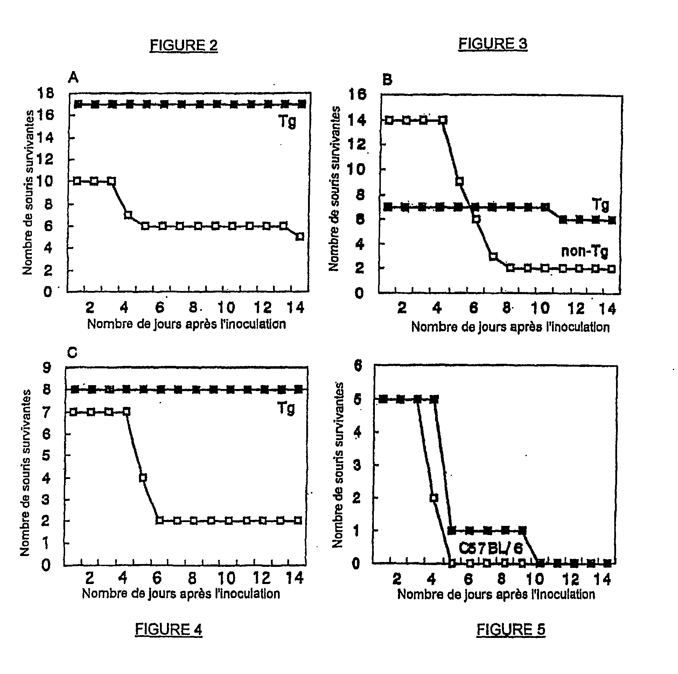

Selon les figures 2, 3, et 4 on a dénombré les souris transgéniques T_{g} respectivement des lignées A B et C et les souris non transgéniques non T_{g} demeurées vivantes jusqu'à 14 jours après l'infection.

On a ainsi pu constater que toutes les lignées de souris transgéniques étaient résistantes au virus HSV-1.

Plus précisément toutes les souris transgéniques des lignées A et C ont survécu à l'inoculation par le virus et sont restées en bonne santé pendant plusieurs mois après l'essai (tableaux 1 et 4).

Seule une souris transgénique de la lignée B, est décédée après l'inoculation par le virus tandis que les six autres ont survécu (tableau 3), mais la lignée B est celle pour laquelle les taux sériques mesurés en HVEMIg étaient les plus faibles.

En revanche, six des sept souris non transgéniques des mêmes portées que les souris transgéniques de la lignée A, 13 des 14 souris non transgéniques des mêmes portées que les souris transgéniques de la lignée B et six des sept souris non transgéniques des mêmes portées que les souris transgéniques de la lignée C ont développé des symptômes tels qu'une paralysie ou sont mortes dans les 14 jours qui ont suivi l'inoculation par le virus HSV-1.

On a recherché l'expression du LAT du virus HSV-1 dans les ganglions trigéminaux des souris survivantes après l'inoculation par la méthode décrite dans les publications.
- Spivak, J. G., and Fraser, N. W. (1987). Detection of herpes virus type 1 transcripts during latent infections in mice. J. Virol., 61, 3841-3847.
- Stevens, J. G., and Cook, M. L. (1971). Latent herpes simplex virus in spinal ganglia of mice. Science 173, 843-845.

On a ainsi mis en oeuvre la méthode de RT-PCR de façon à détecter l'expression du LAT.

Celle-ci a été observée chez les souris non transgéniques ayant développé des symptômes et chez une seule souris transgénique de la lignée B n'ayant pas présenté de symptômes, mais n'a en revanche pas été observée chez toutes les autres souris transgéniques testées ni chez les souris non transgéniques survivantes n'ayant pas développé de symptômes.

Dans le cadre de cette étude on a également effectué un essai de contrôle dans le but de déterminer si les souris transgéniques exprimant la protéine chimérique HVEMIg étaient protégées contre le virus PRV ce alors que la protéine HVEM n'est pas un récepteur fonctionnel pour le virus PRV.

A cet effet, on a inoculé par voie intraveineuse et par une dose correspondant à 10 fois la dose létale (10 LD 50) de virus PRV, des souris transgéniques de la lignée A et des souris non transgéniques de la même portée (tableau 2 et figure 5).

On a ainsi pu constater qu'à l'exception d'une souris transgénique qui a survécu pendant 10 jours toutes les souris sont décédées dans les cinq jours suivant l'inoculation par le virus PRV.

Par suite les souris transgéniques exprimant la protéine chimérique HVEMIg ne se sont pas révélées protégées contre le virus PRV.

Dans le cadre de cette étude, on a également cherché à déterminer si la résistance des souris transgéniques à l'inoculation par le virus HSV-1 ayant pu être constatée *in vivo* s'accompagnait en parallèle d'une résistance des cellules de ces souris une fois isolées.

A cet effet on a inoculé des cultures de fibroblastes embryonnaires de souris transgéniques ou de souris non transgéniques par le virus HSV-1.

On a ensuite dénombré les plages de lyse provoquées par le virus dans la culture cellulaire 5 jours après l'inoculation, et constaté que le nombre de ces plages était nettement plus important dans le cas des fibroblastes de souris non transgéniques que dans le cas de fibroblastes de souris transgéniques (en moyenne 22 plaques par disque de culture pour les souris non transgéniques et 1 plaque par disque de culture pour les souris transgéniques).

On a parallèlement effectué un test similaire pour le virus PRV en inoculant des cultures de fibroblastes embryonnaires issus de souris transgéniques ou de souris non transgéniques par le virus PRV, sans constater de différences notables entre le nombre de plages de lyse observé chez les souris transgéniques et chez les souris non transgéniques.

Ces résultats sont de nature à prouver que la protéine chimérique HVEMIg exprimée par les fibroblastes des souris transgéniques intervient dans l'inhibition de l'adsorption du virus HSV-1 par ces fibroblastes embryonnaires.

Dans un test complémentaire dont les résultats sont illustrés sur le tableau 5 on a recherché si la protéine chimérique HVEMIg présente dans le sérum de souris transgéniques pouvait inhiber l'infection de cultures cellulaires par les virus HSV-1 ou PRV.

Dans ce but, on a recueilli du sérum de souris transgéniques de la lignée C et incubé avec un inoculat de ce sérum le virus HSV-1 ou le virus PRV avant de le mettre en contact avec des cultures de cellules Vero.

On a ainsi pu établir que le sérum de souris transgéniques de la lignée C peut protéger des cellules Vero d'une contamination par le virus HSV-1 mais pas d'une contamination par le virus PRV.

Un test de contrôle effectué avec du sérum de souris non transgéniques n'a au contraire pas permis de constater d'activité antivirale.

On a par ailleurs constaté que le sérum des souris transgéniques de la lignée C ne présente plus d'activité antivirale après qu'il ait été mis en contact avec un sérum polyclonal anti-HVEMIg produit par hyperimmunisation sur lapin pendant 30 minutes à température ambiante.

Ce dernier résultat confirme que les capacités séroneutralisantes du sérum des souris transgéniques sont à attribuer à l'expression de la protéine chimérique.

L'ensemble de ces résultats démontre que l'activité antivirale constatée provient de la protéine chimérique HVEMIg présente dans le sérum des souris transgéniques, mais sont également sans doute associés à l'expression de cette protéine chimérique à la surface des cellules de l'hôte telles les fibroblastes embryonnaires évalués dans ces tests.

Ces résultats confirment l'efficacité antivirale de l'expression in-vivo d'une forme modifiée d'un récepteur membranaire des alphaherpesvirus, ceci en dépit des propriétés immunosuppressives décrites dans le cas de la protéine HveM.
2) des résultats expérimentaux obtenus dans le cadre de l'analyse in vivo de la résistance au virus PRV de souris transgéniques exprimant une protéine chimérique Hvec-Ig.

Selon ces tests on a introduit par transgénèse germinale dans l'ADN de ces souris un transgène codant pour une protéine chimérique constituée du domaine extracellulaire du récepteur porcin HveC du virus PRV et de la portion cristallisable Fc de l'immunoglobuline IgG-1 humaine.

Le domaine extracellulaire porcin HveC a été cloné par RT PCR à partir d'une préparation d'ARN extraite de cellules de porc.

Les amorces utilisées pour la réaction RT PCR étaient 5'-TAACTCGAGCTCTTGGCCTGAAGTTTC-3' et 5'-TTAAGGATCCGAGGAGGTGGTGTCT-3', comme il est décrit et en suivant les conditions proposées dans la publication (Milne, R. S. B., Connolly, S. A., Krummenacher, C., Eisenberg, R. J., and Cohen, G. H. (2001). Porcine HveC, a member of the highly conserved HveC/nectin 1 family, is a functional alphaherpesvirus receptor. Virology 281, 315-32.).

L'ADNc a été inséré dans les sites de restriction Xhol et BamHl d'un plasmide portant la séquence du fragment cristallisable de l'immunoglobuline G1 humaine (comme décrit dans la publication Nakagawa I., Murakami, M., Ijima, K., Chikuma, S., Saito, I., Kanegae, Y., Ishikura, H., Yoshiki, T., Okamoto, H., Kitabatake, A., and Uede, T. (1998). Persistent and secondary adenovirus-mediated hepatic gene expression using adenovirus vector containing CTLA41gG. Human Gene Therapy 9, 1739-1745). Le fragment XbaI/XbaI de ce plasmide contenant l'ADN encodant la fusion HveC-Ig a été isolé, mis à bouts francs et lié à des adaptateurs SalI, ceci afin de l'insérer à son tour, après digestion par Xho1 et Sal1, dans le site de restriction XhoI du vecteur pCXN2 sous le contrôle du promoteur CAG (promoteur de la β actin) connu pour permettre une expression constitutive élevée dans tout type de cellule (Niwa, H., Yamamura, K., and Miyazaki, J. (1991). Efficient selection for high-expression transfectants with a novel eukaryotic vector. Gene 108, 193-200). Le plasmide recombinant ainsi obtenu a été désigné Pcxn2/pHvecIg.

On a créé par micro injection du fragment SalI/SalI de ce plasmide, contenant le facteur de stimulation de la transcription du gène viral CMV IE, le promoteur du gène Beta actin de la poule, la séquence de la protéine HVEMIg et le signal de polyadénylation 3' du locus Beta globin de lapin, dans les pronucléi d'embryons fécondés de souris (souche C57/BL6) six lignées #6, #22, #32, #33, #37, #45 de souris transgéniques chacune issue d'un fondateur indépendant exprimant cette protéine chimérique HveC-Ig.

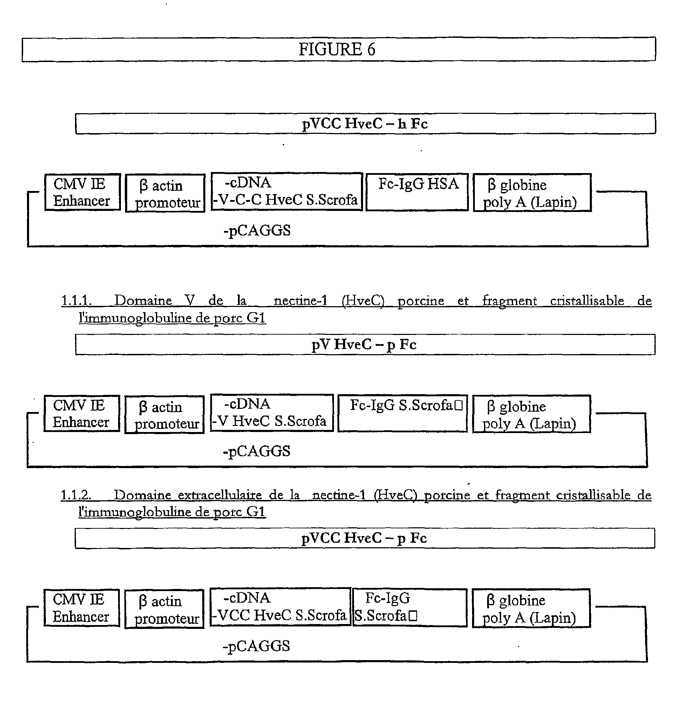

La construction effectuée est représentée schématiquement sur la figure 6.

Les souris transgéniques des six lignées se sont développées normalement et on n'a pas constaté de différences entre les poids de ces souris transgéniques et le poids des souris non transgéniques issues des mêmes portées.

### Epreuves virales par injections intra-péritonéales

Pour déterminer si les souris transgéniques exprimant la protéine chimérique HveC-Ig étaient effectivement protégées contre le virus PRV, on a inoculé dans une première série d'épreuve par voie intrapéritonéale une dose de 500 u.f.p. correspondant à vingt fois la dose létale (20 DL 50) de virus, des souris transgéniques et des souris non transgéniques de contrôle de la même portée.

Selon le tableau 6 on a dénombré les souris transgéniques T_{g} respectivement des lignées #6, #22, #32, #33, #37, #45 et les souris non transgéniques non T_{g} demeurées vivantes jusqu'à 14 jours après l'infection.

On a ainsi pu constater que les 6 lignées de souris transgéniques étaient résistantes au virus PRV dans cette épreuve réputée sévère, avec 100% de survie des animaux transgéniques, à l'exception d'un seul animal pour la lignée #33.

En revanche, seules environ 9% des souris non transgéniques ont survécu, avec une variation selon les épreuves conduites pour chaque lignée.

Ces épreuves ont été confirmées pour les lignées #22 et #32 dans une laboratoire indépendant en utilisant une souche différente de virus PRV.

### Epreuves virales par infection intranasale

On a inoculé dans une deuxième série d'épreuve par voie intranasale une dose de 250 u.f.p. correspondant à dix fois la dose létale (20 DL 50) de virus, des souris transgéniques et des souris non transgéniques de contrôle de la même portée.

Selon le tableau 7 on a dénombré les souris transgéniques T_{g} respectivement des lignées #6, #22, #32, #33, #37 et les souris non transgéniques non T_{g} demeurées vivantes jusqu'à 14 jours après l'infection.

On a ainsi pu constater que les 5 lignées de souris transgéniques étaient relativement résistantes au virus PRV dans cette épreuve réputée sévère, avec environ 70 % de survie des animaux transgéniques.

En revanche, seules environ 10 % des souris non transgéniques ont survécu, avec une variation selon les épreuves conduites pour chaque lignée.

Ces résultats démontrent une action protectrice efficace du transgène permettant l'expression de la protéine chimérique HveC-Ig dans le contexte d'une épreuve sévère chez la souris par voie intra nasale.

Globalement, l'ensemble de ces résultats montre l'efficacité in-vivo de la protection conférée à un mammifère par le procédé conforme à l'invention, basé sur l'inhibition de l'entrée d'un alphaherpesvirus dans la cellule cible. Cette efficacité n'est possible que parce que le procédé proposé permet probablement plusieurs niveaux d'action, dont les mécanismes ne sont pas tous entièrement élucidés : au delà des capacités de ligand des protéines virales gD connues pour les récepteurs HveM, HveC, l'utilisation du domaine extracellulaire seul permet d'isoler ses capacités de récepteur des fonctions biologiques complexes de ces protéines transmembranaires dans leur entrée et notamment de leur aptitude à initier des cascades de signalisation intracellulaires.

Ceci permet a priori d'envisager la surexpression de ce domaine protéique sans pour autant en amplifier la fonction physiologique et permet sans doute l'absence d'effets secondaires observés pour les animaux transgéniques exprimant la protéine chimérique.

Ceci permet probablement dans le contexte d'une infection in vivo :
- une compétition pour la fixation des particules virales infectantes des protéines chimériques en solution ou présentes à la surface des cellules avec les récepteurs membranaires fonctionnels du virus ;
- la neutralisation des particules virales par opsonisation et augmentation de la phagocytose par les macrophages et cellules dendridiques ;
- l'activation de l'immunité cellulaire par stimulation des lymphocytes NK.

L'efficacité du procédé dans le cas des infections expérimentales par voie intra nasale ainsi que (pour HveMIg) la résistance des fibroblastes embryonnaires issus d'animaux transgéniques vont dans le sens d'une inhibition efficace de l'entrée du virus dans les cellules cibles sensibles par la fraction membranaire de la protéine chimérique exprimée, au delà de la capacité séroneutralisante de la fraction soluble sécrétée dans le sérum.

Cette inhibition de l'entrée du virus dans la cellule pourrait être le fait de récepteurs membranaires du virus modifiés, selon un mode dominant, autorisant la fixation du virus à la surface des cellules cibles mais pas son entrée dans le cytoplasme sous une forme infectieuse.

L'efficacité du procédé sur l'entrée du virus et les premiers résultats portant sur la latence virale après l'infection (dans le cas d'HveM et HSV1) permettent de surcroît d'envisager l'absence de latence chez les animaux d'élevage ainsi protégés, ce qui n'est pas nécessairement le cas pour les stratégies vaccinales, et donc par là un contrôle plus efficace des phénomènes de résurgence de l'infection.
3) Des résultats expérimentaux obtenus dans le cadre de l'analyse in vitro de la résistance au virus BHV-1 et PRV de lignées cellulaires transformées par des plasmides exprimant des protéines chimériques construites à partir du domaine extra cellulaire de la protéine HveC porcine et du fragment cristallisable de l'immunoglobuline Ig humaine.

Selon ce test on a préparé des lignées de cellules Vero transformées par des plasmides exprimant une forme soluble de la protéine HveC porcine inhibant l'entrée des virus BHV-1 et PRV puis on a analysé la résistance de ces lignées cellulaires à l'infection par ces virus pour déterminer les propriétés antivirales de la forme soluble de la protéine HveC porcine in vitro.

Pour construire un plasmide exprimant une forme soluble de la protéine HveC porcine (PHveCIg) on a utilisé un vecteur d'expression PCXN2 permettant la transcription d'un ARN messager codant pour le domaine extra cellulaire de la protéine HveC porcine joint au fragment cristallisable Fc de l'immuno globuline IgG1 humaine.

Le domaine extracellulaire porcin HveC a été cloné par RTPCR à partir d'une préparation d'ARN extraite de cellules de porc.

Les amorces utilisées pour la réaction RT PCR étaient 5'-TAACTCGAGCTCTTGGCCTGAAGTTTC-3' et 5'-TTAAGGACCGAGGAGCT-3: comme il est décrit et en suivant les conditions proposées dans la publication (Milne, R. S. B., Connolly, S. A., Krummenacher, C., Eisenberg, R. J., and Cohen, G. H. (2001). Porcine HveC, a member of the highly conserved HveC/nectin 1 family, is a functional alphaherpesvirus receptor. Virology 281, 315-32.).

L'ADNc a été inséré dans les sites de restriction Xhol et BamHl d'un plasmide portant la séquence du fragment cristallisable de l'immunoglobuline G1 humaine (comme décrit dans la publication Nakagawa I., Murakami, M., Ijima, K., Chikuma, S., Saito, I., Kanegae, Y., Ishikura, H., Yoshiki, T., Okamoto, H., Kitabatake, A., and Uede, T. (1998). Persistent and secondary adenovirus-mediated hepatic gene expression using adenovirus vector containing CTLA41gG. Human Gene Therapy 9, 1739-1745). Le fragment XbaI/XbaI de ce plasmide contenant l'ADN encodant la fusion HveC-Ig a été isolé, mis à bouts francs et lié à des adaptateurs SalI, ceci afin de l'insérer à son tour dans le site de restriction XhoI du vecteur pCXN2 sous le contrôle du promoteur CAG (promoteur de la β actin) connu pour permettre une expression constitutive élevée dans tout type de cellule (Niwa, H., Yamamura, K., and Miyazaki, J. (1991). Efficient selection for high-expression transfectants with a novel eukaryotic vector. Gene 108, 193-200). Le plasmide recombinant ainsi obtenu a été désigné Pcxn2/pHvecIg.

On a préparé par transfection avec le plasmide ainsi obtenu des lignées cellulaires transformées de manière stable.

Les lignées cellulaires ainsi transformées ont été cultivées dans des conditions permettant l'accumulation de la protéine chimérique dans le milieu à savoir 24 heures de cultures additionnelles après étalement à subconfluence.

Ces cultures ont ensuite été inoculées par les virus PRV ou BHV-1 avec une multiplicité de 50 u.f.p. par boite de 35 mm de diamètre avec un temps d'incubation d'une heure suivi de deux rinçages au DMEM avant couverture avec du milieu DMEM 0,5 % d'agar.

On a ensuite dénombré les plages de lyse provoquées par le virus dans la culture cellulaire 4 jours après l'inoculation. On a constaté que dans les lignées cellulaires exprimant la protéine chimérique PHveCIg le nombre de plaques de lyse a été notablement réduit par comparaison avec des lignées cellulaires résistantes de contrôle et les lignées Vero quatre jours après l'inoculation.

Les résultats obtenus sont rassemblés dans le tableau 8.

Les lignées cellulaires transformées par le plasmide PCXN2/pHveC Ig sont clairement résistantes à l'affection par les virus PRV et BHV-1..
4) Des résultats expérimentaux obtenus dans le cadre de l'analyse in vitro de la résistance aux virus BHV-1 et PRV de lignées cellulaires transformées par des plasmides exprimant des protéines chimériques construites à partir du domaine extracellulaire de la protéine HveC porcine et du fragment cristallisable de l'immunoglobuline g1 du porc.

Selon ce test on a préparé des lignées de cellules Vero transformées par des plasmides exprimant une forme soluble de la protéine HveC porcine inhibant l'entrée des virus BHV-1 et PRV puis on a analysé la résistance de ces lignées cellulaires à l'infection par ces virus pour déterminer les propriétés antivirales de la forme soluble de la protéine HveC porcine in vitro.

Pour construire un plasmide exprimant une forme soluble de la protéine HveC porcine (PHveCIg) on a utilisé un vecteur d'expression pCXN2 permettant la transcription d'un ARN messager codant pour le domaine extracellulaire de la protéine HveC porcine et le fragment cristallisable Fc de l'immunoglobuline IgG-1 porcine.

Le domaine extracellulaire porcin HveC a été cloné par RT PCR à partir d'une préparation d'ARN extraite de cellules de porc.

Les amorces utilisées pour la réaction RT PCR étaient 5'-TAACTCGAGCTCTTGGCCTGAAGTTTC-3' et 5'-TTAAGGATCCGAGGAG-CAGGTGGTGTCT-3', comme il est décrit et en suivant les conditions proposées dans la publication (Milne, R. S. B., Connolly, S. A., Krummenacher, C., Eisenberg, R. J., and Cohen, G. H. (2001). Porcine HveC, a member of the highly conserved HveC/nectin 1 family, is a functionnal alphaherpesvirus receptor. Virology 281, 315-32.).

L'ADN c ainsi obtenu a été lié en 5' d'un fragment d'ADN c codant pour le fragment cristallisable Fc de l'immunoglobuline Ig porcine en respectant le cadre de lecture original des deux polypeptides.

Le fragment d'ADN c d'immunoglobuline porcine a été cloné à partir d'une préparation d'ARN extraite de tissus lymphoides de porc d'une lignée de type Large White (FHO25) par RT PCR en utilisant en tant qu'amorce TAACTCGAGCTCTTGGCCTGAAGTTTC-3' et 5'-TTAAGGATCCGAGGAG-CAGGTGGTGTCT-3' en suivant les conditions proposées dans la publication Simon Musyoka Mwangi, Thomas J. Stabel*, Marcus E. Kehrli Jr, development of a baculovirus expression system for soluble porcine tumor necrosis factor receptor type 1 and soluble porcine tumor necrosis factor receptor type I-IgG fusion protein, Veterinary Immunology and Immunopathology 86 (2002) 251-254.

L'ADN c résultant de la fusion code pour une protéine chimérique dont la séquence en acides aminés est jointe en annexe (séquence 4).

L'ADN c résultant de la fusion a été inséré dans le site de restriction Xhol du plasmide pCXN2 sous le contrôle du promoteur du gêne bêta actin de la poule associé au facteur de stimulation de la transcription du gène viral CMV IE et la séquence de polyadénylation de la bêta globin de lapin.

Le plasmide résultant ainsi obtenu a été désigné PCXN2/pVCC-pFc.

Une version restreinte de ce plasmide désignée PCXN2/pV-pFc a été construite en utilisant seulement le domaine V de la protéine HveC et le fragment cristallisable Fc de l'immuno-globuline IgG de porc.

La séquence en acides aminés de la protéine chimérique ainsi obtenue est jointe en annexe (séquence 3).

On a préparé par transfection avec le plasmide ainsi obtenu des lignées cellulaires transformées de manière stable.

Les lignées cellulaires ainsi transformées ont été cultivées dans des conditions permettant l'accumulation de la protéine chimérique dans le milieu à savoir 24 heures de cultures additionnelles après étalement à subconfluence.

Ces cultures ont ensuite été inoculées par les virus PRV ou BHV-1 avec une multiplicité de 50 u.f.p. par boite de 35 mm de diamètre avec un temps d'incubation d'une heure suivi de deux rinçages au DMEM avant couverture avec du milieu DMEM 0,5 % d'agar.

On a ensuite dénombré les plages de lyse provoquées par le virus dans la culture cellulaire trois jours après l'inoculation et effectué les constatations rassemblées dans le tableau 9.

Les lignées cellulaires transformées par les deux versions du transgène exprimant une protéine chimérique conforme à l'invention sont clairement résistantes à l'affection par les virus BHV-1 et PRV.

**TABLEAU 1**

| Résistance des souris transgéniques de la lignée A à une inoculation par le virus HSV-1 | | | | | | |
|---|---|---|---|---|---|---|
| Essai | Numéro de l'animal | | Sexe | HVEMIg (ug/ml) | Symptômes | Jour de la mort |
| I | A1 | * | M | 14.8 | - | |
| | A2 | * | M | 10.0 | - | |
| | A3 | * | M | 11.6 | - | |
| | A4 | * | F | 7.6 | - | |
| | A5 | * | F | 8.3 | - | |
| | A6 | * | F | 10.0 | - | |
| | A7 | * | F | 24.6 | - | |
| | A8 | * | F | 8.5 | - | |
| | A9 | * | F | 7.9 | - | |
| | A10 | * | F | 7.3 | - | |
| | | | | | | |
| | L1 | | M | 1.5 | - | |
| | L2 | | M | 1.1 | - | |
| | L3 | | M | 1.6 | + | 4 |
| | L4 | | M | 0.8 | + | 14 |
| | L5 | | M | 1.6 | + | |
| | L6 | | F | 0.6 | + | 5 |
| | | | | | - | |
| II | A11 | * | M | 10.1 | - | |
| | A12 | * | M | 15.9 | - | |
| | A13 | * | M | 10.5 | - | |
| | A14 | * | M | 9.3 | - | |
| | A15 | * | F | 7.3 | - | |
| | A16 | * | F | 14.0 | - | |
| | A17 | * | F | 15.4 | - | |
| | A18 | * | F | 14.9 | - | |
| | | | | | | |
| | L7 | | M | 0.4 | + | 4 |
| | L8 | | M | 0.5 | + | |
| | L9 | | F | 0.5 | + | |

**TABLEAU 2**

| Sensibilité des souris transgéniques de la lignée A à une inoculation par le virus PRV | | | | | | |
|---|---|---|---|---|---|---|
| | Numéro de l'animal | | Sexe | HVEMIg (ug/ml) | Symptômes | Jour de la mort |
| | A19 | * | M | 41.2 | + | 4 |
| | A20 | * | F | 19.4 | + | 10 |
| | A21 | * | F | 29.6 | + | 5 |
| | A22 | * | F | 24.2 | + | 5 |
| | A23 | * | F | 20.2 | + | 5 |
| | | | | | | |
| | C57BL/6 | | M | NT | + | 4 |
| | C57BL/6 | | M | NT | + | 4 |
| | C57BL/6 | | M | NT | + | 5 |
| | C57BL/6 | | M | NT | + | 5 |
| | C57BL/6 | | M | NT | + | 5 |

**TABLEAU 3**

| Résistance des souris transgéniques de la lignée B à une inoculation par le virus HSV-1 | | | | | | | |
|---|---|---|---|---|---|---|---|
| Essai | Numéro de l'animal | | Sexe | HVEMIg (ug/ml) | Symptômes | Jour de la mort | RT-PCR |
| I | B1 | * | M | 8.5 | - | | - |
| | B2 | * | M | 7.8 | - | | - |
| | B3 | * | M | 7.7 | - | | - |
| | B4 | * | F | 8.1 | + | 11 | |
| | | | | | | | |
| | L1 | | M | 0.4 | + | 6 | |
| | L2 | | M 0.7 | | + | 6 | |
| | L3 | | M | 0.5 | + 5 | | |
| | L4 | | M | 0.7 | + 7 | | |
| | L5 | | F | 0.3 | + 5 | | |
| | | | | | | | |
| II | B5 | * | M | 5.0 | - | | + |
| | B6 | * | M | 6.2 | - | | - |
| | B7 | * | F | 4.4 | - | | - |
| | | | | | | | |
| | L6 | | M | 0.6 | - | | |
| | L7 | | M | 0.6 | + | 10 | |
| | L8 | | M | 0.5 | + | 7 | |
| | L9 | | M | 0.6 | + | | + |
| | L10 | | F | 0.4 | + | 7 | |
| | L11 | | F | 0.5 | + | 5 | |
| | L12 | | F | 0.5 | + | 5 | |
| | L13 | | F | 0.5 | + | 5 | |
| | L14 | | F | 0.4 | + | 6 | |

**TABLEAU 4**

| Résistance des souris transgéniques de la lignée C à une inoculation par le virus HSV-1 | | | | | | | |
|---|---|---|---|---|---|---|---|
| Essai | Numéro de l'animal | | Sexe | HVEMIg (ug/ml) | Symptômes | Jour de la mort | RT-PCR |
| | C1 | * | M | 20.4 | - | | - |
| | C2 | * | M | 14.2 | - | | - |
| | C3 | * | F | 19.3 | - | | - |
| | C4 | * | F | 18.5 | - | | - |
| | C5 | * | F | 24.3 | - | | - |
| | C6 | * | F | 21.8 | - | | - |
| | C7 | * | F | 24.0 | - | | - |
| | C8 | * | F | 20.6 | - | | - |
| | | | | | | | |
| | L1 | | M | 1.3 | - | | - |
| | L2 | | M | 0.9 | + | | + |
| | L3 | | M | 1.6 | + | 6 | |
| | L4 | | F | 1.3 | + | 5 | |
| | L5 | | F | 1.4 | + | 5 | |
| | L6 | | F | 1.5 | + | 6 | |
| | L7 | | F | 1.6 | + | 5 | |

Dans les tableaux 1 à 4 les animaux désignés par un * sont des animaux transgéniques alors que les animaux désignés par L sont des animaux non transgéniques de contrôle des mêmes portées.

**TABLEAU 5**

| Neutralisation du virus HVS-1 par la protéine chimérique HVEMIg dans le sérum de souris transgéniques de la lignée C. Inoculation de virus HSV1 sur cellules VERO, après incubation avec des concentrations variables de ce sérum | | |
|---|---|---|
| | Nombre de plages de lyse observées | |
| Serum (HVEMlg ug/ ml) | HSV-1 | PRV |
| (20.4) | 0 | 108.0 ± 8.8 |
| (2.04) | 0 | - |
| (0.20) | 1.7 ± 1.6 | - |
| (0.02) | 34.7 ± 16.2 | - |
| (0.20) + anti HVEMlg | 30.3 ± 6.9 | - |
| Contrôle | 44.0 ± 0 | 107.3 ± 2.9 |

**Tableau 6**

| Epreuves par voie intra péritonéale (20 LD 50). | | | | | | |
|---|---|---|---|---|---|---|
| Lignée | Nombre d'animaux Tg éprouvés | Nombre de contrôles (des mêmes portées) éprouvés | Nombre d'animaux Tg survivant à 14 jours | Nombre de contrôles survivants à 14 jours | % survie animaux transgéniques | % survie des contrôles |
| PHveCIg#6 | 5 | 10 | 5 | 2 | 100 | 20 |
| PHveCIg#22 | 12 | 12 | 12 | 0 | 100 | 0 |
| PHveCIg#32 | 10 | 7 | 10 | 1 | 100 | 14,2 |
| PHveCIg#33 | 7 | 9 | 6 | 0 | 85,7 | 0 |
| PHveCIg#37 | 10 | 4 | 10 | 1 | 100 | 25 |
| PHveCIg#45 | 3 | 12 | 3 | 1 | 100 | 8,3 |

**Tableau 7**

| Epreuves par voie intra nasale (10 LD 50). | | | | | | |
|---|---|---|---|---|---|---|
| Lignée | Nombre d'animaux Tg éprouvés | Nombre de contrôles (des mêmes portées) éprouvés | Nombre d'animaux Tg survivant à 14 jours | Nombre de contrôles survivants à 14 jours | % survie animaux transgéniques | % survie des contrôles |
| PHveCIg#6 | 8 | 7 | 4 | 0 | 50 | 0 |
| PHveCIg#22 | 23 | 21 | 18 | 2 | 78,3 | 9,5 |
| PHveCIg#32 | 10 | 8 | 6 | 1 | 60 | 12,5 |
| PHveCIg#33 | 17 | 8 | 11 | 1 | 64,7 | 12,5 |
| PHveCIg#37 | 10 | 7 | 9 | 1 | 90 | 14,2 |

**Tableau 8**

| Résistance de lignées cellulaires transformées aux alpha herpès virus PRV et BHV-1 | | | |
|---|---|---|---|
| Lignée cellulaire * | PHveCIg | Nombre de plages de lyse observé | |
| | | PRV | BHV-1 |
| A6 | + | 0 | 0 |
| C1 | + | 0 | 0 |
| C2 | - | 55,8 + 4,6 | 75,5 + 3,5 |
| Vero | - | 50,8 + 6,2 | 65,0 + 5,6 |

| | | | |
|---|---|---|---|
| * Dans ce tableau les lignées A6 et C1 sont des lignées cellulaires transformées par le plasmide pCXN2/pHveCIg et exprimant la protéine chimérique PHveCIg alors que la lignée C2 correspond à un témoin négatif de cette transformation n'exprimant pas le transgène et la lignée Vero aux cellules initiales sensibles aux virus et utilisées pour la production des lignées transformées par les différents transgènes. | | | |

**Tableau 9**

| Résistance de lignées cellulaires transformées aux alpha herpès virus PRV et BHV-1 | | |
|---|---|---|
| Lignée cellulaire * | Nombre de plages de lyse observé PRV | |
| | PRV | BHV-1 |
| 3-16 | 0 | 1 |
| V110 | 0 | 6 |
| Vero | 56 | 67 |

| | | |
|---|---|---|
| * Dans ce tableau la lignée 3-16 est une lignée cellulaire transformée par le plasmide p CXN2 / pVCC - pFc et la lignée V110 est une lignée cellulaire transformée par le plasmide p CXN2 / pV - pFc alors que la lignée Vero correspond aux cellules initiales sensibles aux virus et utilisées pour la production de cellules transformées par les différents transgènes. | | |

## Revendications

1. Procédé pour produire un mammifère appartenant à une espèce non humaine rendu résistant par transgénèse germinale à une infection par un alpha herpès virus, pour lequel le polypeptide HveC ou nectin-1 constitue un récepteur fonctionnel
**caractérisé en ce qu'**
on introduit par insertion ou recombinaison homologue dans le génome des cellules constituant la lignée germinale du mammifère, un transgène permettant l'expression d'une protéine chimérique constituée d'une part du domaine extracellulaire de la nectin-1 ou HveC c'est-à-dire des trois domaines protéiques VCC ou d'une forme tronquée de ce domaine extracellulaire consistant en le domaine V, et d'autre part du fragment cristallisable d'une immunoglobuline, dans un système d'expression approprié.

2. Procédé selon la revendication 1,
**caractérisé en ce que**
l'immunoglobuline est une immunoglobuline de type gamma.

3. Procédé selon l'une quelconque des revendications 1 et 2,
**caractérisé en ce que**
la nectin-1 ou HveC et/ou l'immunoglobuline appartiennent à l'espèce homologue.

4. Mammifère appartenant à une espèce non humaine,
**caractérisé en ce qu'**
il a été rendu résistant par transgénèse germinale à une infection par un alpha herpès virus pour lequel le polypeptide HveC ou nectin-1 constitue un récepteur fonctionnel par l'effet de l'expression d'une protéine chimérique constituée d'une part du domaine extracellulaire de la nectin-1 ou HveC c'est-à-dire des trois domaines protéiques VCC ou d'une forme tronquée de ce domaine extracellulaire consistant en le domaine V, de préférence de l'espèce homologue, et d'autre part du fragment cristallisable d'une immunoglobuline, notamment d'une immunoglobuline de type gamma, de préférence de l'espèce homologue.

5. Mammifère selon la revendication 4,
**caractérisé en ce qu'**
il appartient à l'espèce porcine et l'alpha herpès virus est le virus PRV.

6. Mammifère selon la revendication 4,
**caractérisé en ce qu'**
il appartient à l'espèce bovine et l'alpha herpès virus est le virus BHV-1.

7. Mammifère selon l'une quelconque des revendications 4 à 6,
**caractérisé en ce qu'**
il renferme dans le génome de ses cellules, un transgène codant pour une protéine chimérique constituée d'une part du domaine extra cellulaire de la nectin-1 ou HveC c'est-à-dire des trois domaines proteïques VCC ou d'une forme tronquée de ce domaine extra-cellulaire consistant en le domaine V et d'autre part du fragment cristallisable d'une immuno globuline, dans un système d'expression approprié, ce transgène ayant été inséré dans le génome de la lignée germinale de l'un de ses ancêtres.

8. Matériel génétique tel que semence ou ovocyte ou embryon essentiellement dérivé du mammifère selon l'une quelconque des revendications 4 à 7,
**caractérisé en ce qu'**
il contient un transgène permettant l'expression d'une protéine chimérique constituée d'une part du domaine extra cellulaire de la nectin-1 ou HveC, c'est-à-dire des trois domaines proteïques VCC ou d'une forme tronquée de ce domaine extra-cellulaire consistant en le domaine V, et d'autre part du fragment cristallisable d'une immuno globuline dans un système d'expression approprié.

## Claims

1. Method for producing a mammal belonging to a non-human species made resistant by germinal transgenesis to an alpha-herpes virus mediated infection, for which the HveC or nectin-1 polypeptide constitutes a functional receptor, **characterised in that** a transgene enabling expression of a chimeric protein consisting on the one hand of the extracellular domain of nectin-1 or HveC, i.e. of the three VCC protein domains or of a truncated form of this extracellular domain consisting of the V domain, and on the other hand of the crystallisable fragment of an immunoglobulin, is introduced by insertion or homologous recombination into the genome of the cells constituting the germinal line of the mammal, in a suitable expression system.

2. Method according to claim 1, **characterised in that** the immunoglobulin is a gamma type immunoglobulin.

3. Method according to one of claims 1 and 2, **characterised in that** the nectin-1 or HveC and/or immunoglobulin belong to the homologous species.

4. Mammal belonging to a non-human species, **characterised in that** it has been made resistant by germinal transgenesis to an alpha-herpes virus mediated infection for which the HveC or nectin-1 polypeptide constitutes a functional receptor by the effect of the expression of a chimeric protein consisting on the one hand of the extracellular domain of nectin-1 or HveC, i.e. of the three VCC protein domains or of a truncated form of this extracellular domain consisting of the V domain, preferably of the homologous species, and on the other hand of the crystallisable fragment of an immunoglobulin, in particular a gamma type immunoglobulin, preferably of the homologous species.

5. Mammal according to claim 4, **characterised in that** it belongs to the porcine species and the alpha-herpes virus is the PRV virus.

6. Mammal according to claim 4, **characterised in that** it belongs to the bovine species and the alpha-herpes virus is the BHV-1 virus.

7. Mammal according to one of claims 4 to 6, **characterised in that** it contains in the genome of its cells a transgene coding for a chimeric protein consisting on the one hand of the extracellular domain of nectin-1 or HveC, i.e. of the three VCC protein domains or of a truncated form of this extracellular domain consisting of the V domain, and on the other hand of the crystallisable fragment of an immunoglobulin, in a suitable expression system, this transgene having been inserted into the genome of the germinal line of one of its ancestors.

8. Genetic material such as semen or oocyte or embryo essentially derived from the mammal according to one of claims 4 to 7, **characterised in that** it contains a transgene enabling expression of a chimeric protein consisting on the one hand of the extracellular domain of nectin-1 or HveC, i.e. of the three VCC protein domains or of a truncated form of this extracellular domain consisting of the V domain, and on the other hand of the crystallisable fragment of an immunoglobulin, in a suitable expression system.

## Patentansprüche

1. Verfahren zur Herstellung eines Säugers, der einer nicht-menschlichen Spezies angehört und durch Keimbahn-Gentransfer gegen eine Infektion durch ein Alpha-Herpesvirus, für das das Polypeptid HveC oder Nectin-1 ein funktioneller Rezeptor ist, resistent gemacht wurde,
**dadurch gekennzeichnet, dass**
durch Insertion oder homologe Rekombination in das Genom von Zellen, die die Keimbahn des Säugers bilden, ein Transgen in einem geeigneten Expressionssystem eingebracht wird, wobei das Transgen die Expression eines chimären Proteins erlaubt, das einerseits aus der extrazellulären Domäne von Nectin-1 oder HveC, d.h. drei VCC-Proteindomänen oder einer verkürzten Form dieser extrazellulären Domäne, die aus der Domäne V gebildet ist, und andererseits aus dem kristallisationsfähigen Fragment eines Immunglobulins gebildet ist.

2. Verfahren nach Anspruch 1,
**dadurch gekennzeichnet, dass**
das Immunglobulin ein Immunglobulin des Gamma-Typs ist.

3. Verfahren nach einem der Ansprüche 1 und 2,
**dadurch gekennzeichnet, dass**
das Nectin-1 oder HveC und/oder das Immunglobulin zur homologen Art gehören.

4. Säuger, der einer nicht-menschlichen Spezies angehört,
**dadurch gekennzeichnet, dass**
er mittels Keimbahn-Gentransfer gegen eine Infektion durch ein Alpha-Herpesvirus resistent gemacht wurde, für das das Polypeptid HveC oder Nectin-1 ein funktioneller Rezeptor ist, und zwar durch die Wirkung der Expression eines chimären Proteins, das einerseits von der extrazellulären Domäne von Nectin-1 oder HveC bevorzugt homologer Art gebildet ist, d.h. von drei VCC-Proteindomänen oder einer verkürzten Form dieser extrazellulären Domäne, die aus der Domäne V gebildet ist, und andererseits aus dem kristallisationsfähigen Fragment eines Immunglobulins gebildet ist, insbesondere eines Immunglobulins vom Typ Gamma, bevorzugt homologer Art.

5. Säuger nach Anspruch 4,
**dadurch gekennzeichnet, dass**
er der Spezies Schwein angehört und das Alpha-Herpesvirus das PRV-Virus ist.

6. Säuger nach Anspruch 4,
**dadurch gekennzeichnet, dass**
er der Spezies Rind angehört und das Alpha-Herpesvirus das BHV-1-Virus ist.

7. Säuger nach einem der Ansprüche 4 bis 6,
**dadurch gekennzeichnet, dass**
er im Genom seiner Zellen ein Transgen in einem geeigneten Expressionssystem einschließt, wobei das Transgen ein chimäres Protein codiert, das einerseits aus der extrazellulären Domäne von Nectin-1 oder HveC, d.h. drei VCC-Proteindomänen, oder einer verkürzten Form dieser extrazellulären Domäne gebildet ist, die aus der Domäne V gebildet ist, und andererseits aus dem kristallisationsfähigen Fragment eines Immunglobulins gebildet ist, wobei das Transgen in das Genom der Keimbahn eines seiner Vorfahren insertiert wurde.

8. Genetisches Material, wie Sperma, Oocyte oder Embryo, das im Wesentlichen von dem Säuger nach einem der Ansprüche 4 bis 7 stammt,
**dadurch gekennzeichnet, dass**
es ein Transgen in einem geeigneten Expressionssystem enthält, wobei das Transgen die Expression eines chimären Proteins erlaubt, das einerseits aus der extrazellulären Domäne von Nectin-1 oder HveC, d.h. drei VCC-Proteindomänen, oder einer verkürzten Form dieser extrazellulären Domäne, die aus der Domäne V gebildet ist, und andererseits aus dem kristallisationsfähigen Fragment eines Immunglobulins gebildet ist.


### SEQUENCE LISTING

<110> FRANCE HYBRIDES
<120> Procédé pour produire un mammifère rendu résistant à une infection par un alpha herpés virus par transgénèse germinale ainsi que mammifère obtenu par la mise en oeuvre de ce procédé
<130> hvec
<150> Fr02 12775
   <151> 2002-10-15
<160> 4
<170> PatentIn version 3.1
<210> 1
   <211> 440
   <212> PRT
   <213> artificial sequence
<220>
   <223> Protéine artificielle fusionnant le domaine extracellulaire de la protéine HveM de la souris et le fragment cristallisable de d'im munoglobuline G1 humaine
<400> 1
   Met Glu Pro Leu Pro Gly Trp Gly Ser Ala Pro Trp Ser Gln Ala Pro
   1               5                   10                  15
   Thr Asp Asn Thr Phe Arg Leu Val Pro Cys Val Phe Leu Leu Asn Leu
               20                  25                  30
   Leu Gln Arg Ile Ser Ala Gln Pro Ser Cys Arg Gln Glu Glu Phe Leu
           35                  40                  45
   Val Gly Asp Glu Cys Cys Pro Met Cys Asn Pro Gly Tyr His Val Lys
       50                  55                  60
   Gln Val Cys Ser Glu His Thr Gly Thr Val Cys Ala Pro Cys Pro Pro
   65                  70                  75                  80
   Gln Thr Tyr Thr Ala His Ala Asn Gly Leu Ser Lys Cys Leu Pro Cys
   85 90 95
   Gly Val Cys Asp Pro Asp Met Gly Leu Leu Thr Trp Gln Glu Cys Ser
   100 105 110
   Ser Trp Lys Asp Thr Val Cys Arg Cys Ile Pro Gly Tyr Phe Cys Glu
   115 120 125
   Asn Gln Asp Gly Ser His Cys Ser Thr Cys Leu Gln His Thr Thr Cys
   130 135 140
   Pro Pro Gly Gln Arg Val Glu Lys Arg Gly Thr His Asp Gln Asp Thr
   145 150 155 160
   Val Cys Ala Asp Cys Leu Thr Gly Thr Phe Ser Leu Gly Gly Thr Gln
   165 170 175
   Glu Glu Cys Leu Pro Trp Thr Asn Cys Ser Ala Phe Gln Gln Glu Val
   180 185 190
   Arg Arg Gly Thr Asn Ser Thr Asp Thr Thr Cys Ser Ser Asp Pro Glu
   195 200 205
   Glu Pro Lys Ser Cys Asp Lys Thr His Thr Cys Pro Pro Cys Pro Ala
   210 215 220
   Pro Glu Leu Leu Gly Gly Pro Ser Val Phe Leu Phe Pro Pro Lys Pro
   225 230 235 240
   Lys Asp Thr Leu Met Ile Ser Arg Thr Pro Glu Val Thr Cys Val Val
   245 250 255
   Val Asp Val Ser His Glu Asp Pro Glu Val Lys Phe Asn Trp Tyr Val
   260 265 270
   Asp Gly Val Glu Val His Asn Ala Lys Thr Lys Pro Arg Glu Glu Gln
   275 280 285
   Tyr Asn Ser Thr Tyr Arg Val Val Ser Val Leu Thr Val Leu His Gln
   290 295 300
   Asp Trp Leu Asn Gly Lys Glu Tyr Lys Cys Lys Val Ser Asn Lys Ala
   305 310 315 320
   Leu Pro Ala Pro Ile Glu Lys Thr Ile Ser Lys Ala Lys Gly Gln Pro
   325 330 335
   Arg Glu Pro Gln Val Tyr Thr Leu Pro Pro Ser Arg Asp Glu Leu Thr
   340 345 350
   Lys Asn Gln Val Ser Leu Thr Cys Leu Val Lys Gly Phe Tyr Pro Ser
           355                 360                 365
   Asp Ile Ala Val Glu Trp Glu Ser Asn Gly Gln Pro Glu Asn Asn Tyr
       370                 375                 380
   Lys Thr Thr Pro Pro Val Leu Asp Ser Asp Gly Ser Phe Phe Leu Tyr
   385                 390                 395                 400
   Ser Lys Leu Thr Val Asp Lys Ser Arg Trp Gln Gln Gly Asn Val Phe
                   405                 410                 415
   Ser Cys Ser Val Met His Glu Ala Leu His Asn His Tyr Thr Gln Lys
               420                 425                 430
   Ser Leu Ser Leu Ser Pro Gly Lys
           435                 440
<210> 2
   <211> 581
   <212> PRT
   <213> artificial sequence
<220>
   <223> Protéine artificielle fusionnant le domaine extracellulaire (doma ines v-C-C) de la protéine Hvec du porc et le fragment cristallis able de d'immunoglobuline G1 humaine
<400> 2
   Met Ala Arg Met Gly Leu Ala Gly Ala Ala Gly Arg Trp Trp Gly Leu
   1               5                   10                  15
   Ala Leu Gly Leu Thr Ala Phe Phe Leu Pro Gly Ala His Thr Gln Val
               20                  25                  30
   Val Gln Val Asn Asp Ser Met Tyr Gly Phe Ile Gly Thr Asp Val Val
           35                  40                  45
   Leu His Cys Ser Phe Ala Asn Pro Leu Pro Gly Val Lys Ile Thr Gln
       50                  55                  60
   Val Thr Trp Gln Lys Ala Thr Asn Gly Ser Lys Gln Asn Val Ala Ile
   65                  70                  75                  80
   Tyr Asn Pro Ala Met Gly Val Ser Val Leu Ala Pro Tyr Arg Glu Arg
                   85                  90                  95
   Val Glu Phe Leu Arg Pro Ser Phe Thr Asp Gly Thr Ile Arg Leu Ser
   100 105 110
   Arg Leu Glu Leu Glu Asp Glu Gly Val Tyr Ile Cys Glu Phe Ala Thr
   115 120 125
   Phe Pro Ala Gly Asn Arg Glu Ser Gln Leu Asn Leu Thr Val Met Ala
   130 135 140
   Lys Pro Thr Asn Trp Ile Glu Gly Thr Gln Ala Val Leu Arg Ala Lys
   145 150 155 160
   Lys Gly Lys Asp Asp Lys Val Leu Val Ala Thr Cys Thr Ser Ala Asn
   165 170 175
   Gly Lys Pro Pro Ser Val Val Ser Trp Glu Thr His Leu Lys Gly Glu
   180 185 190
   Ala Glu Tyr Gln Glu Ile Arg Asn Pro Asn Gly Thr Val Thr Val Ile
   195 200 205
   Ser Arg Tyr Arg Leu Val Pro Ser Arg Glu Asp His Arg Gln Ser Leu
   210 215 220
   Ala Cys Ile Val Asn Tyr His Met Asp Arg Phe Arg Glu Ser Leu Thr
   225 230 235 240
   Leu Asn Val Gln Tyr Glu Pro Glu Val Thr Ile Glu Gly Phe Asp Gly
   245 250 255
   Asn Trp Tyr Leu Gln Arg Met Asp Val Lys Leu Thr Cys Lys Ala Asp
   260 265 270
   Ala Asn Pro Pro Ala Thr Glu Tyr His Trp Thr Thr Leu Asn Gly Ser
   275 280 285
   Leu Pro Lys Gly Val Glu Ala Gln Asn Arg Thr Leu Phe Phe Arg Gly
   290 295 300
   Pro Ile Asn Tyr Ser Met Ala Gly Thr Tyr Ile Cys Glu Ala Thr Asn
   305 310 315 320
   Pro Ile Gly Thr Arg Ser Gly Gln Val Glu Val Asn Ile Thr Glu Phe
   325 330 335
   Pro Tyr Thr Pro Ser Pro Pro Glu His Ala Asp Pro Glu Glu Pro Lys
   340 345 350
   Ser Cys Asp Lys Thr His Thr Cys Pro Pro Cys Pro Ala Pro Glu Leu
   355 360 365
   Leu Gly Gly Pro Ser Val Phe Leu Phe Pro Pro Lys Pro Lys Asp Thr
   370 375 380
   Leu Met Ile Ser Arg Thr Pro Glu Val Thr Cys Val Val Val Asp Val
   385 390 395 400
   Ser His Glu Asp Pro Glu Val Lys Phe Asn Trp Tyr Val Asp Gly Val
   405 410 415
   Glu Val His Asn Ala Lys Thr Lys Pro Arg Glu Glu Gln Tyr Asn Ser
   420 425 430
   Thr Tyr Arg Val Val Ser Val Leu Thr Val Leu His Gln Asp Trp Leu
   435 440 445
   Asn Gly Lys Glu Tyr Lys Cys Lys Val Ser Asn Lys Ala Leu Pro Ala
   450 455 460
   Pro Ile Glu Lys Thr Ile Ser Lys Ala Lys Gly Gln Pro Arg Glu Pro
   465 470 475 480
   Gln Val Tyr Thr Leu Pro Pro Ser Arg Asp Glu Leu Thr Lys Asn Gln
   485 490 495
   Val Ser Leu Thr Cys Leu Val Lys Gly Phe Tyr Pro Ser Asp Ile Ala
   500 505 510
   Val Glu Trp Glu Ser Asn Gly Gln Pro Glu Asn Asn Tyr Lys Thr Thr
   515 520 525
   Pro Pro Val Leu Asp Ser Asp Gly Ser Phe Phe Leu Tyr Ser Lys Leu
   530 535 540
   Thr Val Asp Lys Ser Arg Trp Gln Gln Gly Asn Val Phe Ser Cys Ser
   545 550 555 560
   Val Met His Glu Ala Leu His Asn His Tyr Thr Gln Lys Ser Leu Ser
   565 570 575
   Leu Ser Pro Gly Lys
   580
<210> 3
   <211> 376
   <212> PRT
   <213> artificial sequence
<220>
<223> Protéine artificielle fusionnant le domaine V de la protéine Hvec du porc et le fragment cristallisable de d'immunoglobuline G1 po reine
<400> 3
   Met Ala Arg Met Gly Leu Ala Gly Ala Ala Gly Arg Trp Trp Gly Leu
   1               5                   10                  15
   Ala Leu Gly Leu Thr Ala Phe Phe Leu Pro Gly Ala His Thr Gln Val
               20                  25                  30
   Val Gln Val Asn Asp Ser Met Tyr Gly Phe Ile Gly Thr Asp Val Val
           35                  40                  45
   Leu His Cys Ser Phe Ala Asn Pro Leu Pro Gly Val Lys Ile Thr Gln
       50                  55                  60
   Val Thr Trp Gln Lys Ala Thr Asn Gly Ser Lys Gln Asn Val Ala Ile
   65                  70                  75                  80
   Tyr Asn Pro Ala Met Gly Val Ser Val Leu Ala Pro Tyr Arg Glu Arg
                   85                  90                  95
   Val Glu Phe Leu Arg Pro Ser Phe Thr Asp Gly Thr Ile Arg Leu Ser
               100                 105                 110
   Arg Leu Glu Leu Glu Asp Glu Gly Val Tyr Ile Cys Glu Phe Ala Thr
           115                 120                 125
   Phe Pro Ala Gly Asn Arg Glu Ser Gln Leu Asn Leu Thr Val Met Gly
       130                 135                 140
   Ser Val Gly Ile His Gln Pro Gln Thr Cys Pro Ile Cys Pro Gly Cys
   145                 150                 155                 160
   Glu Val Ala Gly Pro Ser Val Phe Ile Phe Pro Pro Lys Pro Lys Asp
                   165                 170                 175
   Thr Leu Met Ile Ser Gln Thr Pro Glu Val Thr Cys Val Val Val Asp
               180                 185                 190
   Val Ser Lys Glu His Ala Glu Val Gln Phe Ser Trp Tyr Val Asp Gly
           195                 200                 205
   Val Glu Val His Thr Ala Glu Thr Arg Pro Lys Glu Glu Gln Phe Asn
       210                 215                 220
   Ser Thr Tyr Arg Val Val Ser Val Leu Pro Ile Gln His Gln Asp Trp
   225                 230                 235                 240
   Leu Lys Gly Lys Glu Phe Lys Cys Lys Val Asn Asn Val Asp Leu Pro
                   245                 250                 255
   Ala Pro Ile Thr Arg Thr Ile Ser Lys Ala Ile Gly Gln Ser Arg Glu
   260 265 270
   Pro Gln Val Tyr Thr Leu Pro Pro Pro Ala Glu Glu Leu Ser Arg Ser
   275 280 285
   Lys Val Thr Leu Thr Cys Leu Val Ile Gly Phe Tyr Pro Pro Asp Ile
   290 295 300
   His Val Glu Trp Lys Ser Asn Gly Gln Pro Glu Pro Glu Asn Thr Tyr
   305 310 315 320
   Arg Thr Thr Pro Pro Gln Gln Asp Val Asp Gly Thr Phe Phe Leu Tyr
   325 330 335
   Ser Lys Leu Ala Val Asp Lys Ala Arg Trp Asp His Gly Asp Lys Phe
   340 345 350
   Glu Cys Ala Val Met His Glu Ala Leu His Asn His Tyr Thr Gln Lys
   355 360 365
   Ser Ile Ser Lys Thr Gln Gly Lys
   370 375
<210> 4
   <211> 578
   <212> PRT
   <213> artificial sequence
<220>
   <223> Protéine artificielle fusionnant le domaine extracellulaire (doma ines v-c-C) de la protéine Hvec du porc et le fragment cristallis able de d'immunoglobuline G1 porcine
<400> 4
   Met Ala Arg Met Gly Leu Ala Gly Ala Ala Gly Arg Trp Trp Gly Leu
   1 5 10 15
   Ala Leu Gly Leu Thr Ala Phe Phe Leu Pro Gly Ala His Thr Gln Val
   20 25 30
   Val Gln Val Asn Asp Ser Met Tyr Gly Phe Ile Gly Thr Asp Val Val
   35 40 45
   Leu His Cys Ser Phe Ala Asn Pro Leu Pro Gly Val Lys Ile Thr Gln
   50 55 60
   Val Thr Trp Gln Lys Ala Thr Asn Gly Ser Lys Gln Asn Val Ala Ile
   65                  70                  75                  80
   Tyr Asn Pro Ala Met Gly Val Ser Val Leu Ala Pro Tyr Arg Glu Arg
                   85                  90                  95
   Val Glu Phe Leu Arg Pro Ser Phe Thr Asp Gly Thr Ile Arg Leu Ser
               100                 105                 110
   Arg Leu Glu Leu Glu Asp Glu Gly Val Tyr Ile Cys Glu Phe Ala Thr
           115                 120                 125
   Phe Pro Ala Gly Asn Arg Glu Ser Gln Leu Asn Leu Thr Val Met Ala
       130                 135                 140
   Lys Pro Thr Asn Trp Ile Glu Gly Thr Gln Ala Val Leu Arg Ala Lys
   145                 150                 155                 160
   Lys Gly Lys Asp Asp Lys Val Leu Val Ala Thr Cys Thr Ser Ala Asn
                   165                 170                 175
   Gly Lys Pro Pro Ser Val Val Ser Trp Glu Thr His Leu Lys Gly Glu
               180                 185                 190
   Ala Glu Tyr Gln Glu Ile Arg Asn Pro Asn Gly Thr Val Thr Val Ile
           195                 200                 205
   Ser Arg Tyr Arg Leu Val Pro Ser Arg Glu Asp His Arg Gln Ser Leu
       210                 215                 220
   Ala Cys Ile Val Asn Tyr His Met Asp Arg Phe Arg Glu Ser Leu Thr
   225                 230                 235                 240
   Leu Asn Val Gln Tyr Glu Pro Glu Val Thr Ile Glu Gly Phe Asp Gly
                   245                 250                 255
   Asn Trp Tyr Leu Gln Arg Met Asp Val Lys Leu Thr Cys Lys Ala Asp
               260                 265                 270
   Ala Asn Pro Pro Ala Thr Glu Tyr His Trp Thr Thr Leu Asn Gly Ser
           275                 280                 285
   Leu Pro Lys Gly Val Glu Ala Gln Asn Arg Thr Leu Phe Phe Arg Gly
       290                 295                 300
   Pro Ile Asn Tyr Ser Met Ala Gly Thr Tyr Ile Cys Glu Ala Thr Asn
   305                 310                 315                 320
   Pro Ile Gly Thr Arg Ser Gly Gln Val Glu Val Asn Ile Thr Glu Phe
                   325                 330                 335
   Pro Tyr Thr Pro Ser Pro Pro Glu His Gly Ser Val Gly Ile His Gln
   340 345 350
   Pro Gln Thr Cys Pro Ile Cys Pro Gly Cys Glu Val Ala Gly Pro Ser
   355 360 365
   Val Phe Ile Phe Pro Pro Lys Pro Lys Asp Thr Leu Met Ile Ser Gln
   370 375 380
   Thr Pro Glu Val Thr Cys Val Val Val Asp Val Ser Lys Glu His Ala
   385 390 395 400
   Glu Val Gln Phe Ser Trp Tyr Val Asp Gly Val Glu Val His Thr Ala
   405 410 415
   Glu Thr Arg Pro Lys Glu Glu Gln Phe Asn Ser Thr Tyr Arg Val Val
   420 425 430
   Ser Val Leu Pro Ile Gln His Gln Asp Trp Leu Lys Gly Lys Glu Phe
   435 440 445
   Lys Cys Lys Val Asn Asn Val Asp Leu Pro Ala Pro Ile Thr Arg Thr
   450 455 460
   Ile Ser Lys Ala Ile Gly Gln Ser Arg Glu Pro Gln Val Tyr Thr Leu
   465 470 475 480
   Pro Pro Pro Ala Glu Glu Leu Ser Arg Ser Lys Val Thr Leu Thr Cys
   485 490 495
   Leu Val Ile Gly Phe Tyr Pro Pro Asp Ile His Val Glu Trp Lys Ser
   500 505 510
   Asn Gly Gln Pro Glu Pro Glu Asn Thr Tyr Arg Thr Thr Pro Pro Gln
   515 520 525
   Gln Asp Val Asp Gly Thr Phe Phe Leu Tyr Ser Lys Leu Ala Val Asp
   530 535 540
   Lys Ala Arg Trp Asp His Gly Asp Lys Phe Glu Cys Ala Val Met His
   545 550 555 560
   Glu Ala Leu His Asn His Tyr Thr Gln Lys Ser Ile Ser Lys Thr Gln
   565 570 575
   Gly Lys